# FASCICULE DE BREVET EUROPEEN

(11) **EP 1 037 563 B1**
(45) Date de publication et mention de la délivrance du brevet: **03.03.2004**
(21) Numéro de dépôt: 98958324.0
(22) Date de dépôt: 04.12.1998
(51) Int. Cl.: A61B 17/70

(54) **SYSTEME D'OSTEOSYNTHESE**
SYSTEM ZUR OSTEOSYNTHESE
OSTEOSYNTHESIS SYSTEM

(30) Priorité: 16.12.1997 FR 9715912
(43) Date de publication de la demande: 27.09.2000
(73) Titulaire: Razian, Hassan, 94240 l'Hay les Roses (FR)
(72) Inventeur: Razian, Hassan, 94240 l'Hay les Roses (FR)
(74) Mandataire: Flavenot, Bernard
(86) Numéro de dépôt international: PCT/FR1998/002617
(87) Numéro de publication internationale: WO 1999/030627

(56) Documents cités:
- EP-A- 0 328 883
- EP-A- 0 611 116
- EP-A- 0 637 437
- WO-A-93/01772
- DE-U- 9 218 381
- FR-A- 2 728 454
- US-A- 5 290 288
- US-A- 5 569 247

## Description

La présente invention concerne les systèmes d'ostéosynthèse permettant de relier des os, par exemple des vertèbres pour corriger des défauts pathologiques de la colonne vertébrale comme des hernies discales, des fractures vertébrales, des spondylolisthesis, cyphoses, lordoses, scolioses, instabilités vertébrales, etc.

On connaît déjà, par le FR-A-2 728 454, un système d'ostéosynthèse rachidienne destiné au réglage et au maintien positionnel des vertèbres pour corriger des défauts pathologiques de la colonne vertébrale, comportant des vis pédiculaires implantées dans des vertèbres et un élément de liaison reliant les vis pédiculaires entre elles et avec les vertèbres, l'élément de liaison comportant une partie mâle s'engageant dans une partie femelle, la partie femelle comprenant au moins une partie relativement flexible de manière à former un élément de liaison semi-flexible, des moyens de réglage de la flexibilité et de l'amplitude du mouvement en flexion, extension, inflexion, traction, compression et rotation de l'élément de liaison et des moyens de blocage longitudinal et de flexibilité en traction-compression de l'élément de liaison.

Le système d'ostéosynthèse rachidienne décrit ci-dessus présente des avantages et répond aux demandes des praticiens qui doivent soigner des patients dont la colonne vertébrale présente des défauts pathologiques comme ceux cités ci-dessus. Mais il présente aussi des inconvénients, notamment pour sa fabrication de façon industrielle.

On connait ausi un dispositif permettant de maintenir deux os l'un par rapport à l'autre, comme celui qui est décrit dans le EP-A-0 328 883. Ce dispositif est essentiellement constitué de deux parties de tige filetées dans des sens de filetage opposés et montées en coopération avec un manchon fileté intérieurement d'un double filetage complémentaire respectivement des deux filetages des deux parties de tige, les deux parties de tige étant terminées par des orifices circulaires aptes à coopérer avec des vis pédiculaires.

Ce dispositif n'est en fait utilisé, et utilisable, que pour ajuster la distance entre les centres des orifices circulaires et, quelle que soit la position des deux parties de tige, il ne présente qu'une seule possibilité de rigidité.

On connaît aussi, du US-A-5 569 247, un système de vis pédiculaires comportant une tige à filetage osseux, une tige à filetage mécanique et une tête interposée entre ces deux tiges dont la structure permet d'ajuster la valeur angulaire entre les axes de ces deux tiges de façon qu'elles puissent plus facilement coopérer avec une plaque longitudinale apte à réaliser une ostéosynthèse.

En fait aucun des dispositifs décrits ci-dessus ne permet, à lui seul, de réaliser une liaison entre deux os en vue d'obtenir leur ostéosynthèse, qui soit, selon la volonté du praticien, rigide ou semi-rigide.

La présente invention a ainsi pour but de réaliser un système d'ostéosynthèse d'une structure relativement simple, qui permet d'obtenir, selon les désirs du praticien, une liaison osseuse rigide ou semi-rigide, moyennant simplement une modification de son agencement qui ne pose aucune difficulté au praticien.

Plus précisément, la présente invention a pour objet un système d'ostéosynthèse comportant :
- une plaque longitudinale de liaison entre au moins deux os, ladite plaque longitudinale comportant au moins deux parties, chaque partie de plaque comportant des moyens d'emboîtement respectivement femelles et mâles associables les uns dans les autres selon l'axe longitudinal de la plaque, et
- des moyens pour solidariser ladite plaque longitudinale avec les deux os, cesdits moyens pour solidariser ladite plaque longitudinale avec les deux os comportant au moins deux orifices oblongs réalisés dans ladite plaque suivant son axe longitudinal respectivement dans les deux dites parties de plaque et au moins deux couples "vis pédiculaire-écrou", chaque couple étant apte à coopérer avec un orifice, chaque vis pédiculaire comportant une tige à filetage osseux, une tige à filetage mécanique et une tête de vissage réunissant les deux tiges respectivement à filetage osseux et à filetage mécanique,
caractérisé par le fait que les moyens d'emboîtement respectivement femelles et mâles sont constitués par un filetage dit "femelle" situé sur la paroi d'une partie en creux réalisée dans l'une des deux parties de plaque et par un filetage dit "mâle" réalisé sur une tige en saillie solidaire de l'autre partie de plaque, cedit filetage mâle étant complémentaire du filetage femelle, et par le fait que la longueur du filetage femelle est supérieure à la longueur de la tige en saillie.

D'autres caractéristiques et avantages de la présente invention apparaîtront au cours de la description suivante donnée en regard des dessins annexés à titre illustratif, mais nullement limitatif, dans lesquels:
La figure 1 représente, sous forme schématique, une vue partiellement écorchée d'un mode de réalisation d'une plaque de liaison entrant dans la constitution du système d'ostéosynthèse selon l'invention,
Les figures 2A et 2B représentent, sous forme schématique, un mode de réalisation de vis pédiculaires entrant dans la constitution du système d'ostéosynthèse selon l'invention,
La figure 3 représente, sous forme schématique, un système d'ostéosynthèse selon l'invention utilisant des vis pédiculaires comme celles illustrées sur les figures 2A et 2B,
Les figures 4A à 4C représentent respectivement trois modes de réalisation d'un écrou autobloquant apte à coopérer avec des vis pédiculaires entrant dans la constitution du système d'ostéosynthèse selon l'invention, notamment celles illustrées sur les figures 2A et 2B, et
Les figures 5 et 6 représentent respectivement deux modes de réalisation de moyens entrant dans la constitution du système d'ostéosynthèse selon l'invention, pour relier une plaque de liaison se présentant sous la forme d'une tige avec des parties osseuses de vertèbres.

Le Demandeur tient à préciser que ces figures ne représentent que des exemples de mode de réalisation de l'objet selon l'invention, mais qu'il peut exister d'autres modes de réalisation qui répondent à la définition de cette invention.

Il précise en outre que, lorsque, selon la définition de l'invention, l'objet de l'invention comporte "au moins" un élément ayant une fonction donnée, le mode de réalisation décrit peut comporter plusieurs de ces éléments.

Il précise aussi que, si les modes de réalisation de l'objet selon l'invention tel qu'illustrés comportent plusieurs éléments de fonction identique et si, dans la description, il n'est pas spécifié que l'objet selon cette invention doit obligatoirement comporter un nombre particulier de ces éléments, l'objet de l'invention pourra être défini comme comportant "au moins" un de ces éléments.

Il est tout d'abord précisé que le système d'ostéosynthèse selon l'invention comporte une plaque longitudinale 1 de liaison entre au moins deux os, qui peut prendre différentes formes, par exemple celle d'une bande ou analogue, ou celle d'une tige de forme sensiblement cylindrique ou analogue, ou une combinaison de ces deux formes, etc.

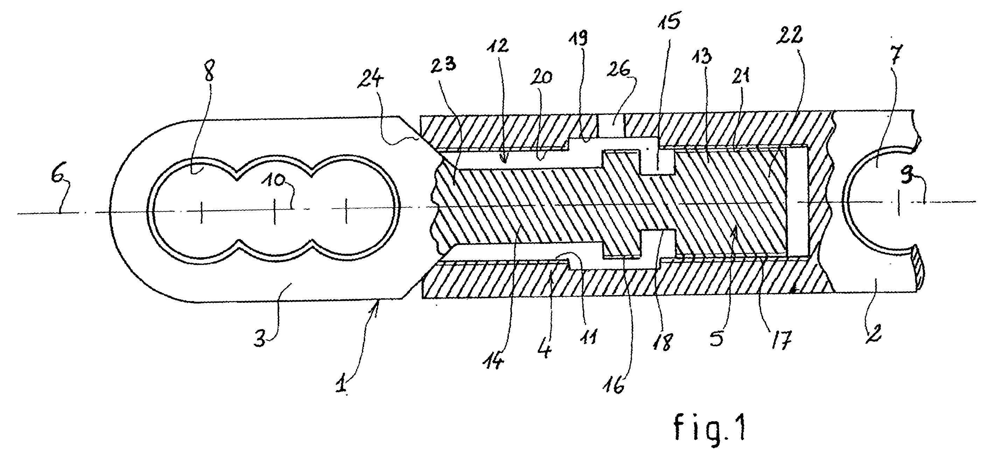

Dans le cas du mode de réalisation du système d'ostéosynthèse illustré sur la figure 1, la plaque longitudinale 1 de liaison entre au moins deux os, par exemple deux vertèbres, comporte au moins deux parties 2, 3, chaque partie de plaque comportant des moyens d'emboîtement respectivement femelles 4 et mâles 5 associables les uns dans les autres selon une direction donnée 6 et des moyens pour solidariser les parties de plaque respectivement avec les deux vertèbres.

Dans ce même mode réalisation, les moyens d'emboîtement respectivement femelles 4 et mâles 5 sont constitués par un filetage dit "femelle" 11 situé sur la paroi d'une partie en creux 12 réalisée dans l'une 2 des deux parties de plaque et par un filetage dit "mâle" 13 réalisé sur une tige en saillie 14 solidaire de l'autre partie de plaque 3, ce filetage mâle étant complémentaire du filetage femelle pour qu'il puisse s'y visser.

Avantageusement, la longueur du filetage femelle 11 est supérieure à la longueur de la tige en saillie 14 pour que l'on puisse obtenir, en bout de course du vissage de la tige en saillie dans la partie en creux, un blocage complet des deux parties de plaque 2 et 3 l'une dans l'autre comme il sera décrit ci-après.

Le système d'ostéosynthèse comporte alors aussi une gorge circulaire 15 pour déterminer, dans le filetage mâle 13, deux portions de filetage mâle 16 et 17, la valeur du diamètre du fond 18 de la gorge circulaire 15 étant inférieure à celle du diamètre de la tige en saillie 14. La partie de tige en saillie délimitée au niveau du fond 18 de la gorge 15 définit ainsi, dans la tige en saillie, un point de faiblesse qui favorise la localisation d'une rupture de sécurité en deux morceaux de la plaque de liaison dans le cas où, pour quelque raison que ce soit, elle serait soumise à des efforts trop importants.

Le système d'ostéosynthèse comporte en outre une rainure circulaire 19 pour déterminer, dans le filetage femelle 11, deux portions de filetage femelle 20, 21, la largeur de cette rainure 19 étant avantageusement supérieure à la longueur de la portion de filetage mâle 16 qui est la plus éloignée de l'extrémité libre 22 de la tige en saillie 14 par rapport à la partie de plaque 3 dont cette tige en saillie est solidaire par son autre extrémité 23.

De façon avantageuse, la paroi de la partie en creux 12 comporte une percée traversante de part en part 26 qui débouche dans la rainure circulaire 19. Cette percée traversante permet le passage d'un outil adapté, par exemple un outil de matage, pour déformer le filetage de la partie de filetage mâle 16 définie ci-dessus, dans le but d'empêcher que les deux parties de plaque 2 et 3 ne se dévissent et d'empêcher la séparation totale de ces deux parties de plaque dans le cas d'une rupture au niveau de la gorge circulaire 15 définie ci-avant.

De préférence, le système d'ostéosynthèse comporte en outre des moyens 24 pour renforcer l'association des deux parties de plaque 2, 3 quand elles sont emboîtées l'une dans l'autre en fond de course du vissage de la tige en saillie 14 dans la partie en creux 12, c'est-à-dire pour renforcer la rigidification de la plaque de liaison lorsqu'elle est dans une configuration comme celle qui est illustrée sur la figure 1. Ces moyens de rigidification 24 peuvent prendre différentes formes. Ils peuvent notamment être constitués par l'un des moyens suivants: emboîtement mâle-femelle conique, filetages complémentaires ou analogues, etc. Dans le mode de réalisation illustré sur la figure 1, ils sont constitués par un emboîtement conique mâle-femelle.

La structure de plaque de liaison décrite ci-dessus permet d'obtenir des plaques de différentes configurations.

Il est par exemple possible de réaliser une plaque de liaison présentant une semi-rigidité. Ce résultat est obtenu en arrêtant le vissage de la tige en saillie 14 dans la partie en creux 12 avant que les moyens de rigidification 24 de la plaque de liaison ne coopèrent entre eux. De cette façon, la plaque de liaison présente une certaine souplesse en même temps que la sécurité d'une possibilité de rupture au niveau du point de faiblesse constitué par la gorge circulaire 15 réalisée dans la tige en saillie comme défini ci-avant.

Mais il est aussi possible de réaliser une plaque de liaison présentant une relativement forte rigidité, comme dans la configuration représentée sur la figure 1. Ce résultat est obtenu en vissant à fond la tige en saillie dans la partie en creux, jusqu'à la coopération des moyens de rigidification 24, c'est-à-dire, dans le cas du mode réalisation illustré, de façon à coincer en force l'emboîtement conique 24. Cette réalisation permet en outre d'ajuster la flexibilité de la plaque.

Comme mentionné ci-avant, le système d'ostéosynthèse selon l'invention comporte une plaque longitudinale 1 de liaison entre au moins deux os, comme des vertèbres, et des moyens pour solidariser la plaque longitudinale avec les deux os.

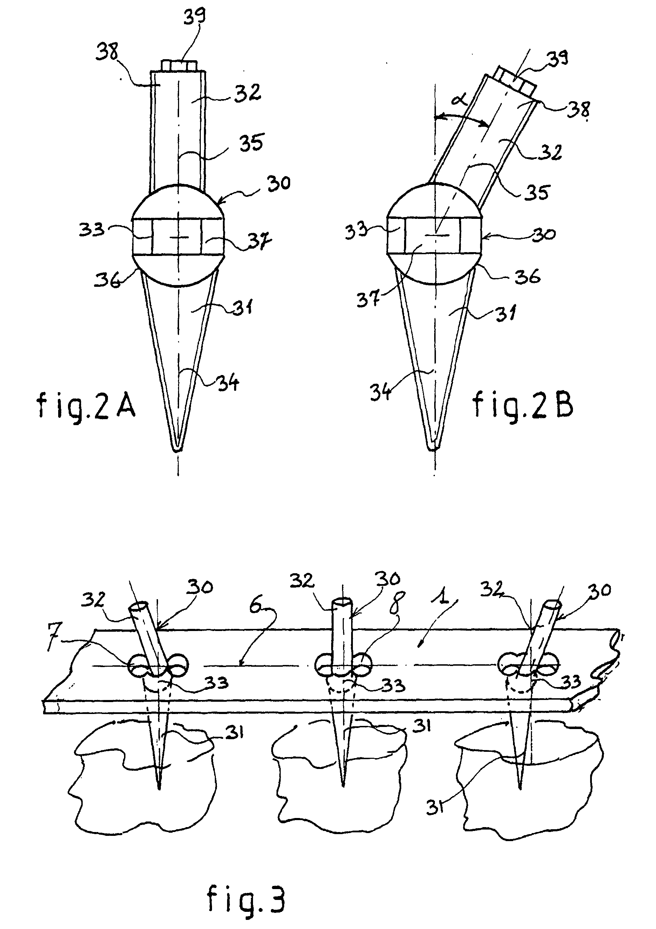

Dans une réalisation avantageuse, ces moyens sont constitués par au moins deux orifices oblongs 7, 8, figure 1, réalisés dans la plaque et au moins deux couples "vis pédiculaire-écrou" 30, 40, figures 2A, 2B, 3 et 4A à 4C, chaque couple étant apte à coopérer avec un orifice oblong et les axes longitudinaux 9, 10 des orifices oblongs étant sensiblement confondus avec l'axe longitudinal 6 de la plaque.

En se reportant plus particulièrement aux figures 2A et 2B, chaque vis pédiculaire 30 comporte une tige à filetage osseux 31, une tige à filetage mécanique 32 et une tête de vissage 33 réunissant les deux tiges à filetage osseux 31 et à filetage mécanique 32.

Selon une caractéristique importante de la présente invention, le système d'ostéosynthèse comporte en combinaison un premier type et un second type de vis pédiculaire, les axes 34, 35 des deux tiges respectivement à filetage osseux 31 et à filetage mécanique 32 étant confondus pour le premier type de vis pédiculaire représenté sur la figure 2A, et les deux axes 34, 35 faisant entre eux un angle α non nul pour le second type représenté sur la figure 2B.

Pour obtenir une bonne coopération entre les vis pédiculaires et la plaque longitudinale 1 par l'intermédiaire des orifices oblongs 7, 8, ceux-ci présentent avantageusement la forme bien connue en elle-même représentée schématiquement sur la figure 1 et telle que décrite et illustrée dans la demande de brevet français ci-dessus référencée.

La tête de vissage 33 affecte sensiblement une forme générale sphérique 36 et comporte des moyens 37 de réception d'un outil apte à l'entraîner en rotation pour visser la tige à filetage osseux 31 dans une partie osseuse. Ces moyens 37 sont par exemple constitués par la partie médiane de la tête de vissage conformée en écrou, par exemple six pans, pour permettre à une clé plate ou analogue d'enserrer la tête et pouvoir ainsi faire pivoter la vis pédiculaire.

De façon préférentielle, l'extrémité libre 38 de la tige à filetage mécanique 32 comporte des moyens 39 pour pouvoir manipuler la vis pédiculaire en vue par exemple de lui faire subir des rotations autour de l'axe 34 de la tige à filetage osseux 31 quand elle est déjà en grande partie vissée dans un os, de façon à orienter l'axe 35 de la tige à filetage mécanique 32 dans le but qui sera défini ci-après en regard notamment de la figure 3. Ces moyens 39 sont par exemple constitués par une forme en saillie polygonale située à cette extrémité 38, apte à recevoir une clé du type à tube ou analogue.

La structure de ces vis pédiculaires leur permet de coopérer plus facilement avec une plaque de liaison 1 comportant au moins trois orifices oblongs, comme illustré sur la figure 3. En effet, il est concevable qu'il est très difficile d'implanter trois vis pédiculaires dans des os comme des vertèbres de façon que les axes de vissage 34 des tiges à filetage osseux 31 soient parallèles et contenus dans un même plan et à une distance parfaitement définie les uns des autres.

Avec le système d'ostéosynthèse selon l'invention, le praticien va pouvoir implanter les trois vis pédiculaires 30 (fig. 3) de façon qu'elles prennent le mieux possible leur emplacement pour pouvoir coopérer avec une plaque de liaison 1 comme celle décrite ci-dessus. Le praticien réalise ensuite la plaque de liaison en associant des parties de plaque comme décrites ci-avant de façon qu'elle ait la rigidité voulue et que les trois orifices oblongs soient sensiblement aptes à entourer les trois tiges à filetage mécanique 32.

Bien entendu, il est certain que ces trois tiges à filetage mécanique ne pourront généralement pas être parfaitement alignées. Aussi, pour compenser, le praticien a-t-il avantage à utiliser, par exemple, deux vis pédiculaires du second type (fig. 2B) et une vis du premier type (fig. 2A) entre les deux autres.

Dans le cas illustré sur la figure 3, il visse chaque vis pédiculaire du second type jusqu'à ce que l'extrémité 38 de sa tige 32 vienne sensiblement en regard de l'orifice oblong correspondant, puis il place la plaque de liaison en faisant passer chacune des trois tiges 32 par l'orifice oblong qui lui correspond. Si la tige 32 de l'une des vis pédiculaires du second type ne pénètre pas facilement dans l'orifice, le praticien peut visser la vis pédiculaire jusqu'à ce que l'extrémité 38 de la tige 32 vienne en regard de l'orifice qui lui est destiné.

La plaque de liaison est glissée le long des tiges 32 jusqu'au contact des têtes de vissage 33, tout en continuant, si nécessaire, à tourner les vis pédiculaires au moyen d'une clé à tube ou analogue.

Lorsque la plaque de liaison est parfaitement mise en place, des écrous 40 sont vissés sur les tiges à filetage mécanique 32 pour solidariser le tout.

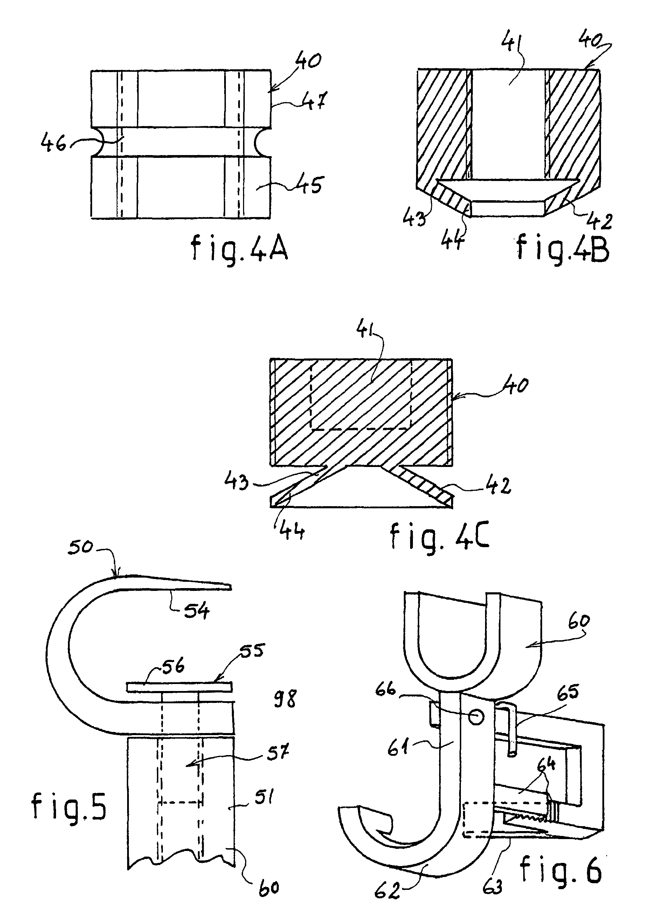

Dans une réalisation préférentielle, ces écrous 40 sont des écrous autobloquants. Les figures 4A, 4B et 4C en représentent différents modes de réalisation.

La figure 4A représente un premier mode de réalisation d'un écrou autobloquant 40 qui comporte une partie formant écrou de serrage 45 et une rainure de faiblesse 46 réalisée dans la surface latérale extérieure 47 de la partie formant écrou de serrage, cette rainure 46 réalisant une ligne de faiblesse de façon que, sous l'action d'un couple de torsion exercé sur ses deux extrémités, la partie formant écrou de serrage se casse en deux parties pour former un système "écrou/contre-écrou" avec un moyen de blocage entre eux.

Le moyen de blocage entre l'écrou et le contre-écrou est constitué par la partie de matière qui se trouve au niveau du fond de la rainure 46 et qui vient se sertir dans le filetage de la tige à filetage mécanique 32 sous l'action de la rotation, l'une sur l'autre, des deux parties de l'écrou de serrage 45 après qu'elles aient été séparées.

Les figures 4 B et 4C représentent un autre mode de réalisation d'un écrou autobloquant qui comporte une partie formant écrou de serrage 41 et une rondelle 42 du type conique solidaire, par l'une de ses bases 43, de l'une des faces de la partie formant écrou de serrage 41. Cette rondelle est centrée sur l'axe de la partie formant écrou de serrage et son autre base 44 est d'un diamètre sensiblement égal à celui du filetage de la partie formant écrou de serrage 41. La figure 4B représente un écrou à filetage interne, tandis que la figure 4C représente un écrou à filetage externe.

Ces écrous sont utilisés de la façon suivante: ils sont vissés sur la tige à filetage mécanique 32 et, lorsqu'ils viennent au contact par exemple de la plaque de liaison 1, la rondelle conique 42 s'écrase et, par sa base 44, vient se sertir dans le filetage de la tige 32. L'écrou et la tige sont ainsi liés de façon permanente, ce qui interdit à l'écrou de se dévisser sous l'action d'effets extérieurs comme des efforts répétitifs, des vibrations, etc.

Comme mentionné ci-avant, la plaque longitudinale de liaison 1 peut prendre différentes formes. Il peut notamment être avantageux de lui donner la forme d'une barre cylindrique ou analogue et d'éviter l'utilisation de vis pédiculaires pour sa fixation sur les os.

Dans ce but, les moyens pour solidariser la plaque 1 avec un os, par exemple une vertèbre, comportent au moins un crochet 50 d'accrochage à une partie osseuse et des moyens 51 pour associer le crochet 50 avec la plaque en forme de barre.

Dans le mode de réalisation illustré sur la figure 5, le crochet 50 comporte une pièce sensiblement en forme de "U" 54 et des moyens 55 pour pincer au moins une partie osseuse d'une vertèbre dans cette pièce en "U" 54. Avantageusement, ces moyens 55 sont constitués par un patin 56 et par des moyens 57 pour monter ce patin 56 en translation à l'intérieur de la pièce en "U" 54 suivant une direction sensiblement perpendiculaire au deux branches du "U".

Les moyens 57 qui permettent de translater le patin 56 par rapport aux branches du "U" peuvent prendre différentes formes, par exemple un vérin à vis ou analogue commandable manuellement ou avec un outil adapté. Ces moyens ne présentant pas de difficulté majeure de mise en oeuvre par un homme du métier, ne seront pas plus amplement décrits ici dans l'unique souci de ne pas compliquer la présente description.

La figure 6 représente une autre réalisation avantageuse des moyens pour solidariser la plaque 1 avec un os. Dans ce mode de réalisation, ces moyens sont constitués par une tête de fixation 60 apte à coopérer avec la plaque et des moyens de pince 61 solidaires de cette tête de fixation 60 et aptes à emprisonner une partie de l'os. Ces moyens de pince 61 comportent par exemple au moins deux pattes 62, 63 montées pivotantes l'une par rapport à l'autre autour d'un axe 66 et des moyens de crémaillère 64 pour les lier l'une avec l'autre dans une position donnée quand elles pincent l'os, de façon à éviter qu'elles ne se désolidarisent de cet os. Pour faciliter le positionnement de ces moyens de pince sur une partie osseuse, les moyens de pince 61 comportent des moyens 65 pour exercer une force élastique entre les deux pattes 62, 63 pour tendre à les rapprocher automatiquement l'une de l'autre. Ces moyens sont par exemple constitués par un ressort solidaire, par l'une de ses extrémités, de l'une des deux pattes et s'appuyant sur l'autre par son autre extrémité.

## Revendications

1. Système d'ostéosynthèse comportant : une plaque longitudinale (1) de liaison entre au moins deux os, ladite plaque longitudinale comportant:au moins deux parties (2, 3), chaque partie de plaque comportant des moyens d'emboîtement respectivement femelles (4) et mâles (5) associables les uns dans les autres selon l'axe longitudinal (6) de la plaque, et des moyens pour solidariser ladite plaque longitudinale avec les deux os, cesdits moyens pour solidariser ladite plaque longitudinale (1) avec les deux os comportant au moins deux orifices (7, 8) oblongs réalisés dans ladite plaque suivant son axe longitudinal (6) respectivement dans les deux dites parties de plaque (2, 3) et au moins deux couples "vis pédiculaire-écrou" (30, 40), chaque couple étant apte à coopérer avec un orifice, chaque vis pédiculaire (30) comportant une tige à filetage osseux (31), une tige à filetage mécanique (32) et une tête de vissage (33) réunissant les deux tiges respectivement à filetage osseux et à filetage mécanique, **caractérisé par le fait, que** les moyens d'emboîtement respectivement femelles (4) et mâles (5) sont constitués par un filetage dit "femelle" (11) situé sur la paroi d'une partie en creux (12) réalisée dans l'une (2) des deux parties de plaque et par un filetage dit "mâle" (13) réalisé sur une tige en saillie (14) solidaire de l'autre partie de plaque (3), cedit filetage mâle étant complémentaire du filetage femelle, et **par le fait que** la longueur du filetage femelle (11) est supérieure à la longueur de la tige en saillie (14).

2. Système d'ostéosynthèse selon la revendication 1, **caractérisé par le fait qu'**il comporte une gorge circulaire (15) pour déterminer, dans le filetage mâle (13), deux portions de filetage mâle (16, 17), la valeur du diamètre du fond (18) de la gorge circulaire (15) étant inférieure à celle du diamètre de la tige en saillie (14).

3. Système d'ostéosynthèse selon la revendication 2, **caractérisé par le fait qu'**il comporte une rainure circulaire (19) pour déterminer dans le filetage femelle (11) deux portions de filetage femelle (20, 21), la largeur de cette rainure (19) étant supérieure à la longueur de la portion de filetage mâle (16) la plus éloignée de l'extrémité libre (22) de la tige en saillie (14) par rapport à la partie de plaque (3) dont cette tige en saillie est solidaire par son autre extrémité (23).

4. Système d'ostéosynthèse selon l'une des revendications 1 à 3, **caractérisé par le fait qu'**il comporte des moyens (24) pour rigidifier entre elles les deux parties de plaque (2, 3) quand elles sont emboîtées l'une dans l'autre en fond de course du vissage de la tige en saillie dans la partie en creux.

5. Système d'ostéosynthèse selon la revendication 4, **caractérisé par le fait que** les moyens (24) pour rigidifier entre elles les deux parties de plaque quand elles sont emboîtées l'une dans l'autre en fond de course du vissage de la tige en saillie dans la partie en creux sont constitués par l'un des moyens suivants: emboîtement mâle-femelle conique, filetages complémentaires.

6. Système d'ostéosynthèse selon l'une des revendications 1 à 5, **caractérisé par le fait que** l'écrou (40) de chaque couple "vis pédiculaire-écrou) est apte à coopérer par vissage avec la tige à filetage mécanique (32) de la vis pédiculaire et est constitué par un écrou autobloquant.

7. Système d'ostéosynthèse selon la revendication 6, **caractérisé par le fait que** ledit écrou autobloquant comporte une partie formant écrou de serrage (41) et une rondelle (42) du type conique solidaire, par l'une de ses bases (43), de l'une des faces de la partie formant écrou de serrage (41), ladite rondelle étant centrée sur l'axe de la partie formant écrou de serrage et son autre base (44) étant d'un diamètre sensiblement égal à celui du filetage de la partie formant écrou de serrage (41).

8. Système d'ostéosynthèse selon la revendication 6, **caractérisé par le fait que** ledit écrou autobloquant (40) comporte une partie formant écrou de serrage (45) et une rainure de faiblesse (46) réalisée dans la surface latérale extérieure (47) de ladite partie formant écrou de serrage (45), cette rainure (46) réalisant une ligne de faiblesse de façon que, sous l'action d'un couple de torsion exercé sur ses deux extrémités, la partie formant écrou de serrage se casse en deux parties pour former un système "écrou/contre-écrou" avec un moyen de blocage entre eux.

9. Système d'ostéosynthèse selon l'une des revendications 1 à 8, **caractérisé par le fait qu'**il comporte deux premier et second types de vis pédiculaire, les axes (34, 35) des deux tiges respectivement à filetage osseux (31) et à filetage mécanique (32) étant confondus pour le premier type de vis pédiculaire (fig. 2A) et faisant entre eux un angle (α) non nul pour le second type (fig. 2B).

10. Système d'ostéosynthèse selon l'une des revendications 1 à 9, **caractérisé par le fait que** ladite tête de vissage (33) de chaque vis pédiculaire affecte sensiblement une forme générale sphérique (36) et comporte des moyens (37) de réception d'un outil apte à l'entraîner en rotation.

## Patentansprüche

1. Osteosynthese-System, das eine longitudinale Verbindungsplatte zwischen wenigstens zwei Knochen umfaßt, die ihrerseits enthält: wenigstens zwei Teile (2, 3), wovon jedes Buchseneinpaßmittel (4) bzw. Steckeinpaßmittel (5) besitzt, die längs der longitudinalen Achse (6) der Platte miteinander verbunden werden können, und Mittel zum Befestigen der longitudinalen Platte an den zwei Knochen, wobei diese Mittel für die Befestigung der longitudinalen Platte (1) an den zwei Knochen wenigstens zwei Langlöcher (7, 8), die in der Platte längs ihrer longitudinalen Achse (6) in diesen beiden Plattenteilen (2, 3) ausgebildet sind, und wenigstens zwei "Pedikelschrauben/Muttern"-Paare (30, 40) umfassen, wobei jedes Paar mit einem Loch zusammenwirken kann, wobei jede Pedikelschraube (30) einen Stift (31) mit Knochengewinde, einen Stift (32) mit mechanischem Gewinde und einen Schraubenkopf (33), der die beiden Stifte mit Knochengewinde bzw. mit mechanischem Gewinde miteinander vereinigt, aufweist, **dadurch gekennzeichnet, daß** die Buchseneinpaßmittel (4) bzw. die Steckeinpaßmittel (5) durch ein sogenanntes "Innengewinde" (11), das sich an der Wand eines hohlen Teils (12) befindet, das in einem (2) der zwei Plattenteile ausgebildet ist, und durch ein sogenanntes "Außengewinde" (13), das an einem vorstehenden Stift (14) ausgebildet ist, der mit dem anderen Plattenteil (3) verbunden ist, gebildet sind, wobei das Außengewinde zu dem Innengewinde komplementär ist, und dadurch, daß die Länge des Innengewindes (11) größer als die Länge des vorstehenden Stifts (14) ist.

2. Osteosynthese-System nach Anspruch 1, **dadurch gekennzeichnet, daß** es eine kreisförmige Nut (15) aufweist, um in dem Außengewinde (13) zwei Außengewindeabschnitte (16, 17) festzulegen, wobei der Wert des Durchmessers des Bodens (18) der kreisförmigen Nut (15) kleiner als jener des Durchmesser des vorstehenden Stifts (14) ist.

3. Osteosynthese-System nach Anspruch 2, **dadurch gekennzeichnet, daß** es eine kreisförmige Rinne (19) aufweist, die in dem Innengewinde (11) zwei Innengewindeabschnitte (20, 21) festlegt, wobei die Breite dieser Rinne (19) größer ist als die Länge des Außengewindeabschnitts (16), der von dem freien Ende (22) des vorstehenden Stifts (14) in bezug auf das Plattenteil (3), mit dem dieser vorstehende Stift über sein anderes Ende (23) verbunden ist, am weitesten entfernt ist.

4. Osteosynthese-System nach einem der Ansprüche 1 bis 3, **dadurch gekennzeichnet, daß** es Mittel (24) umfaßt, die die beiden Plattenteile (2, 3) untereinander versteifen, wenn diese am Ende der Schraubbewegung des vorstehenden Stifts in den hohlen Teil ineinander eingepaßt sind.

5. Osteosynthese-System nach Anspruch 4, **dadurch gekennzeichnet, daß** die Mittel (24), die die beiden Plattenteile miteinander versteifen, wenn diese am Ende der Schraubbewegung des vorstehenden Stifts in den hohlen Teil ineinander eingepaßt sind, durch eines der folgenden Mittel gebildet sind: konische Steck/Buchsen-Einpassung, komplementäre Gewinde.

6. Osteosynthese-System nach einem der Ansprüche 1 bis 5, **dadurch gekennzeichnet, daß** die Mutter (40) jedes "Pedikelschrauben-Mutter"-Paars durch Verschrauben mit dem Stift (32) mit mechanischem Gewinde der Pedikelschraube zusammenwirken kann und durch eine selbstblockierende Mutter gebildet ist.

7. Osteosynthese-System nach Anspruch 6, **dadurch gekennzeichnet, daß** die selbstblockierende Mutter ein eine Klemmutter (41) bildendes Teil und eine konische Scheibe (42), die über eine ihrer Grundflächen (43) mit einer der Flächen des die Klemmutter (41) bildenden Teils verbunden ist, umfaßt, wobei die Scheibe auf die Achse des die Klemmutter bildenden Teils zentriert ist und wobei ihre andere Grundfläche (44) einen Durchmesser besitzt, der im wesentlichen gleich jenem des Gewindes des die Klemmutter (41) bildenden Teils ist.

8. Osteosynthese-System nach Anspruch 6, **dadurch gekennzeichnet, daß** die selbstblockierende Mutter (40) ein eine Klemmutter (45) bildendes Teil und eine Schwächungsrinne (46), die in der äußeren seitlichen Oberfläche (47) des die Klemmutter (45) bildenden Teils verwirklicht ist, umfaßt, wobei diese Rinne (46) eine Schwächungslinie darstellt, derart, daß das die Klemmutter bildende Teil unter der Wirkung eines auf seine beiden Enden ausgeübten Torsionsmoments in zwei Teile zerbricht, um ein "Mutter/Gegenmutter"-System mit dazwischen befindlichem Blockierungsmittel zu bilden.

9. Osteosynthese-System nach einem der Ansprüche 1 bis 8, **dadurch gekennzeichnet, daß** es zwei erste und zweite Typen von Pedikelschrauben umfaßt, wobei die Achsen (34, 35) der zwei Stifte mit Knochengewinde (31) bzw. mit mechanischem Gewinde (32) für den ersten Typ der Pedikelschraube (Fig. 2A) zusammenfallen und für den zweiten Typ (Fig. 2B) untereinander einen von null verschiedenen Winkel (α)bilden.

10. Osteosynthese-System nach einem der Ansprüche 1 bis 9, **dadurch gekennzeichnet, daß** der Schraubenkopf (33) jeder Pedikelschraube im wesentlichen eine im allgemeine sphärische Form (36) besitzt und Mittel (37) für die Aufnahme eines Werkzeugs umfaßt, das ihn in Drehung versetzen kann.

## Claims

1. An osteosynthesis system comprising: a longitudinal plate (1) for linking together at least two bones, said longitudinal plate comprising at least two portions (2, 3), each portion of the plate having respective female and male mutual engagement means (4, 5) that can be associated one in the other along the longitudinal axis (6) of the plate, and means for securing said longitudinal plate with the two bones, said means for securing said longitudinal plate (1) with the two bones comprising at least two oblong orifices (7, 8) made in said plate along its longitudinal axis (6) in respective ones of said two plate portions (2, 3) and at least two "pedicular screw and nut" pairs (30, 40), each pair being suitable for co-operating with one orifice, each pedicular screw (30) having a shank with a bone thread (31), a shank with a machine thread (32), and a screw head (33) uniting the two shanks having the bone thread and the machine thread, respectively, the system being **characterized by** the fact that the male and female mutual engagement means (4, 5) are constituted by a "female" thread (11) situated on the wall of a hollow portion (12) made in one of the plate portions (2) and by a "male" thread (13) made on a projecting shank (14) secured to the other plate portion (3), said male thread being complementary to the female thread, and by the fact that the length of the female thread (11) is greater than the length of the projecting shank (14).

2. An osteosynthesis system according to claim 1, **characterized by** the fact that it includes a circular groove (15) for determining two male thread portions (16, 17) in the male thread (13), the value of the diameter of the bottom (18) of the circular groove (15) being less than the value of the diameter of the projecting shank (14).

3. An osteosynthesis system according to claim 2, **characterized by** the fact that it includes a circular groove (19) for determining two female thread portions (20, 21) in the female thread (11), the width of said groove (19) being greater than the length of the male thread portion (16) that is further away from the free end (22) of the projecting shank (14) relative to the plate portion (3) to which said shank is secured via its other end (23).

4. An osteosynthesis system according to any one of claims 1 to 3, **characterized by** the fact that it includes means (24) for mutually stiffening the two plate portions (2, 3) when they are engaged one in the other at the end of the screw-tightening stroke of the projecting shank in the hollow portion.

5. An osteosynthesis system according to claim 4, **characterized by** the fact that the means (24) for mutually stiffening the two plate portions when they are engaged one in the other at the end of the screw-tightening stroke of the projecting shank in the hollow portion are constituted by one of the following means: conical male-female engagement; complementary threads.

6. An osteosynthesis system according to any one of claims 1 to 5, **characterized by** the fact that the nut (40) of each "pedicular screw and nut" pair is suitable for screw co-operation with the machine thread shank (32) of the pedicular screw and is constituted by a self-locking nut.

7. An osteosynthesis system according to claim 6, **characterized by** the fact that the self-locking nut has a clamping nut forming portion (41) and a conical type washer (42) secured via one of its bases (43) to one of the faces of the clamping nut forming portion (41), said washer being centered on the axis of said clamping nut forming portion and its other base (44) being of a diameter substantially equal to that of the thread of the clamping nut forming portion (41 ).

8. An osteosynthesis system according to claim 6, **characterized by** the fact that said self-locking nut (40) has a clamping nut forming portion (45) and a groove of weakness (46) formed in the outer side surface (47) of said clamping nut forming portion (45), said groove (46) forming a line of weakness such that under the action of torque exerted on its two ends, the clamping nut forming portion breaks into two portions in order to form a "nut and lock nut" system with blocking means between them.

9. An osteosynthesis system according to any one of claims 1 to 8, **characterized by** the fact that it has two types of pedicular screw, a first type and a second type, the axes (34, 35) of the bone thread shank (31) and of the machine thread shank (32) coinciding in the first type of pedicular screw (Figure 2A) and being at a mutual non-zero angle (α) in the second type of pedicular screw (Figure 2B).

10. An osteosynthesis system according to any one of claims 1 to 9, **characterized by** the fact that said screw-tightening head (33) of each pedicular screw is generally substantially spherical in shape (36) and has means (37) for receiving a tool suitable for turning it.
